(19) Europäisches Patentamt
European Patent Office
Office européen des brevets

(11) Veröffentlichungsnummer: **0 201 778**
**B1**

(12) # EUROPÄISCHE PATENTSCHRIFT

(45) Veröffentlichungstag der Patentschrift: **28.12.88**

(51) Int. Cl.⁴: **C 07 C 125/073**, C 07 C 127/19, C 08 F 20/36, A 61 K 6/08

(21) Anmeldenummer: **86105718.0**

(22) Anmeldetag: **25.04.86**

(54) **(Meth)-Acrylsäureester und ihre Verwendung.**

(30) Priorität: **07.05.85 DE 3516257**

(43) Veröffentlichungstag der Anmeldung: **20.11.86 Patentblatt 86/47**

(45) Bekanntmachung des Hinweises auf die Patenterteilung: **28.12.88 Patentblatt 88/52**

(84) Benannte Vertragsstaaten: **AT BE CH DE FR GB IT LI NL SE**

(56) Entgegenhaltungen:
**EP-A- 0 041 145**
**DE-A- 3 106 367**
**GB-A- 1 321 760**
**US-A- 3 726 886**
**US-A- 3 979 426**
**US-A- 4 356 296**
**US-A- 4 424 395**

**ORGANIC COATINGS AND PLASTICS CHEMISTRY, Band 42, 23.-28. März 1980, Houston, Texas, J.R. GRIFFITH et al. "The synthesis of fluorinated acrylics via fluoro tertiary alcohols", pp. 204-207**

(73) Patentinhaber: **BAYER AG, Konzernverwaltung RP Patentabteilung, D-5090 Leverkusen 1 Bayerwerk (DE)**

(72) Erfinder: **Reiners, Jürgen, Dr., Carl-Rumpff-Strasse 57, D-5090 Leverkusen 1 (DE)**
Erfinder: **Winkel, Jens, Dr., Hahnenweg 6, D-5000 Köln 80 (DE)**
Erfinder: **Klauke, Erich, Dr., Eichendorffweg 8, D-5068 Odenthal (DE)**
Erfinder: **Süling, Carlhans, Dr., Carl-Leverkus-Strasse 10, D-5068 Odenthal (DE)**
Erfinder: **Podszun, Wolfgang, Dr., Wolfskaul 4, D-5000 Köln 80 (DE)**

**EP 0 201 778 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue fluorhaltige Acrylsäure- und Methacrylsäureester, im folgenden (Meth)-Acrylsäureester genannt, und ihre Herstellung. Die neuen Verbindungen können als Monomere für die Anwendung im Dentalbereich eingesetzt werden.

Fluorhaltige Phenylcarbinol-acrylate wie 1,1,1,3,3,3-Hexafluor-2-Phenyl-2-acryloyloxy-propan sind aus Org. Coat. Plast. Chem. 42, 204–207, (1980) bekannt. Ähnlich aufgebaute (Meth)acrylsäureester, wie 1,3-Bis-(2-meth)acryloxyl-oxy-1,1,1,3,3,3-hexafluoropropyl-2)-5-perfluoralkylbenzol und ihre Verwendung auf dem Dentalgebiet werden in der US-PS-4356296 beschrieben. Durch die Trifluormethylgruppen werden die Carbinole acidifiziert und die daraus hergestellten Carbinolester zeichnen sich durch eine verminderte Hydrolysenbeständigkeit aus. Dadurch ist ihre Verwendbarkeit als Dentalmonomere eingeschränkt.

Weiterhin ist die Verwendung von 1,1,5-Trihydro-octafluoro-pentyl-methacrylat in Zahnfüllmassen in J. Dent. Res. 58, 1181–1186 (1979) beschrieben.

Monomere dieses Typs liefern Dentalmaterialien mit niedrigem mechanischen Eigenschaftsniveau.

Es wurden neue (Meth)-Acrylsäureester der Formel

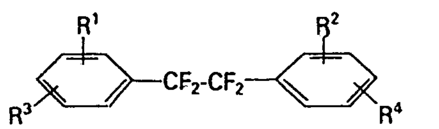

(I),

gefunden, in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

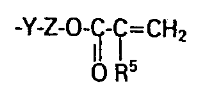

stehen, wobei

$R^5$ für Wasserstoff oder Methyl steht,

Y ein zweibindiges Brückenglied aus der Gruppe

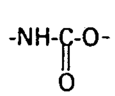 oder 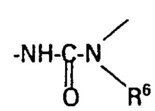 und

wobei

$R^6$ für Wasserstoff, Niederalkyl oder Phenyl steht, bedeutet, und

Z eine geradkettige oder verzweigte Kohlenwasserstoffkette mit 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist.

Im Rahmen der vorliegenden Erfindung können die Substituenten im allgemeinen folgende Bedeutung haben.

Alkyl mit 1 bis 4 Kohlenstoffatomen kann sowohl geradkettig als auch verzweigt sein. Beispielsweise seien die folgenden Alkylreste genannt: Methyl, Ethyl, Propyl, Isopropyl, Butyl und Isobutyl.

Niederalkyl kann einen geradkettigen oder verzweigten Kohlenwasserstoffrest mit 1 bis etwa 4 Kohlenstoffatomen bedeuten. Beispielsweise seien die folgenden Niederalkylreste genannt: Methyl, Ethyl, Propyl, Isopropyl, Butyl, Isobutyl, Pentyl, Isopentyl, Hexyl und Isohexyl.

Z ist im allgemeinen eine geradkettige oder verzweigte Kohlenwasserstoffkette mit 2 bis 10 Kohlenstoffatomen, die durch gegebenenfalls 1 bis 4 Methacrylat- oder Acrylatreste substituiert ist. Beispielsweise seien die folgenden Kohlenwasserstoffketten genannt: Ethylen, Propylen, 2-(Meth)acryloyloxy-1,3-propylen, 3-(Meth)-acryloyloxy-1,2-propylen, 2-(Meth)acryloyloxymethyl-2-ethyl-1,3-propylen und 2,2-Bis(meth)acryloyloxymethyl-1,3-propylen.

Die neue (Meth)-Acrylsäureester sind farblos, schwerflüchtig und ergeben nach Polymerisation transparente Kunststoffe.

Sie lassen sich besonders gut in Abdichtungsmitteln, Klebestoffen und vorzugsweise Dentalmaterialien, wie Zahnfüllmassen und Beschichtungsmitteln, verwenden. Die so erhaltenen Materialien zeichnen sich durch eine überraschend grosse Widerstandsfähigkeit gegenüber physikalischer und chemischer Beanspruchung aus. In besonderem Masse sind Härte und Bruchfestigkeit gegenüber üblichen, zu diesem Zweck eingesetzten Materialien, verbessert. Besonders hervorzuheben sind die günstigen Oberflächeneigenschaften und geringe Wasseraufnahme der mit den neuen (Meth)-Acrylsäureestern erhaltenen Polymerisate.

Bevorzugte (Meth)-Acrylsäureester sind Verbindungen der Formel

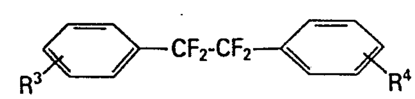

(II)

in der

$R^3$ und $R^4$ gleich oder verschieden sind und für einen der Reste

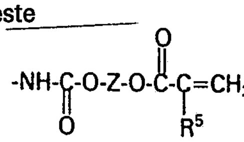

stehen, wobei

$R^5$ Wasserstoff oder Methyl bedeutet, und

Z eine geradkettige oder verzweigte Kohlenwasserstoffkette von 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist.

Die Substituenten $R^3$ und $R^4$ stehen vorzugsweise in 3,3'- oder 3,4'- oder 4,4'-Stellung im 1,2-Diphenyl-tetrafluorethan. Besonders günstige mechanische Werte und niedrige Monomerviskositäten werden erhalten, wenn Gemisch von Stellungsisomeren eingesetzt werden. Am besten eignen sich Gemische mit überwiegendem Anteil an 3,3'- oder 3,4'-Isomeren.

Bei den Substituenten $R^3$ und $R^4$ handelt es sich um Methacrylat-Gruppen enthaltende Alkyloxycarbonylaminoreste, die man z. B. durch Umsetzung entsprechender Isocyanatgruppen mit Methacrylat-Gruppen enthaltenden Hydroxylverbindungen erhalten kann. Bevorzugt sind z. B. Hydroxyethyl(meth)acrylate, 2-Hydroxypropyl(meth)-acrylate, Trimethylolpropandi(meth)acrylat, Propantrioldi(meth)acrylate sowie Pentaerythrittri-(meth)acrylat, Dipentaerythritpenta(meth)acrylat.

Auch Hydroxyverbindungen, die sowohl Acrylatals auch Methacrylatgruppen enthalten, sind gut geeignet. Besonders bevorzugte Hydroxylverbindungen sind 2-Hydroxypropylmethacrylat und Propantrioldimethacrylat (Gemisch von 1,2- und 1,3-Dimethacrylat) und Hydroxy-methacryloyloxyacryloyloxypropan (Gemisch von 1,2- und 1,3-Diester).

Beispielsweise seien die folgenden (Meth)-Acrylsäureester genannt:

Tabelle 1

1 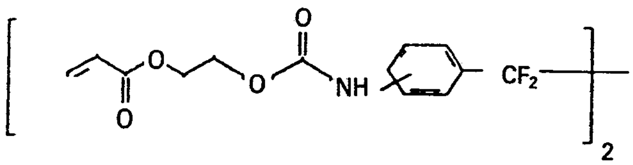

2 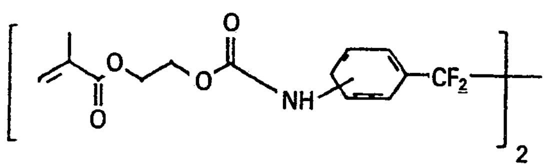

3 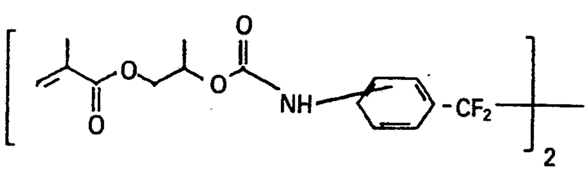

4 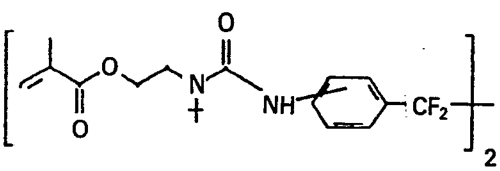

5 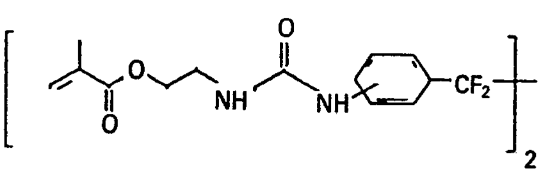

6 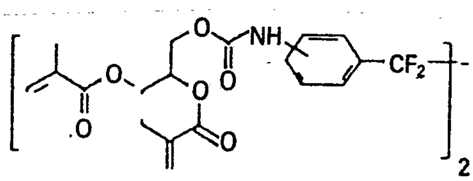

7 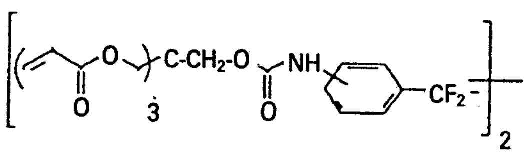

Tabelle 1 (Fortsetzung)

8 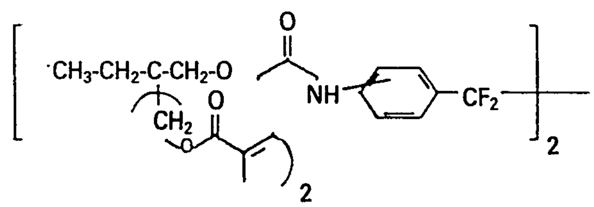

9 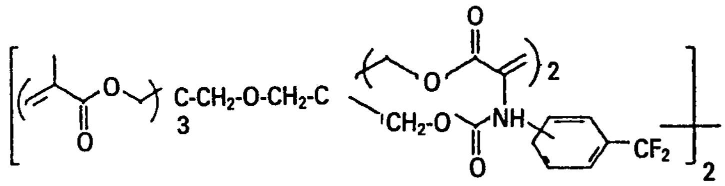

10 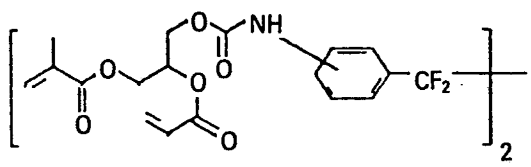

11 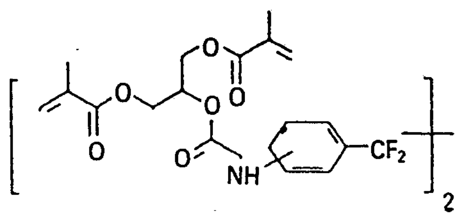

Es wurde auch ein Verfahren zur Herstellung der erfindungsgemässen (Meth)-Acrylsäureester gefunden, das dadurch gekennzeichnet ist, dass man ein 1,2-Bis-(isocyanatophenyl)-tetrafluorethan der Formel

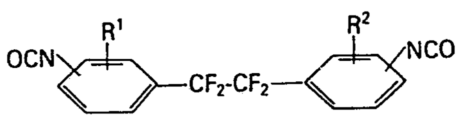 (III),

in der

$R^1$ und $R^2$ die obengenannte Bedeutung haben, mit (Meth)-Acrylsäure-Derivaten der Formel

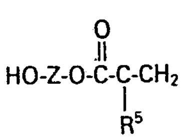 oder 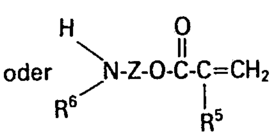 (IV),

in denen

Z eine geradkettige oder verzweigte Kohlenwasserstoffkette mit 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist,

$R^5$ Wasserstoff oder Methyl und

$R^6$ Wasserstoff, Niederalkyl oder Phenyl bedeuten,

in einem inerten Lösungsmittel in Gegenwart eines Katalysators im Temperaturbereich von 20 bis 100°C umsetzt.

Die Herstellung der Isocyanatverbindungen der Formel III kann beispielsweise gemäss Houben-Weyl, Methoden der Organischen Chemie, Band VIII, S. 119ff. (G. Thieme Verlag, Stuttgart 1952), durch Umsetzung der entsprechenden Diaminoverbindungen mit Phosgen durchgeführt werden [siehe auch: Zh. Obshch. Khim. 32 (9), 3035–3039 (1962), und 35 (9), 1612–1620 (1965) und RA 168274 (1963)].

Die Umsetzung der Diisocyanate gemäss Formel III zu den erfindungsgemässen (Meth)acrylsäureestern erfolgt vorzugsweise unter Wasserausschluss in einem inerten Lösungsmittel. Beispiele für geeignete Lösungsmittel sind: Chloroform, Tetrahydrofuran, Dioxan, Methylenchlorid, Toluol, Acetonitril und Frigene. Bevorzugte Lösungsmittel sind Chloroform, Tetrahydrofuran, Frigen 113 und Acetonitril.

Die Umsetzung wird im allgemeinen im Temperaturbereich von 20 bis 100 °C, vorzugsweise 30 bis 70 °C durchgeführt.

Zur Beschleunigung der Umsetzung werden vorzugsweise zinnhaltige Katalysatoren wie Dibutylzinndilaurat oder Zinn(II)octoat verwendet. Andere geeignete Katalysatoren sind Verbindungen mit tert. Aminogruppe und Titanverbindungen. Im allgemeinen wird der Katalysator in einer Menge von 0,01 bis 2,5 Gew.-%, bevorzugt von 0,1 bis 1,5 Gew.-%, bezogen auf die Gesamtmenge der Reaktanden, eingesetzt.

Das erfindungsgemässe Verfahren wird im allgemeinen vorteilhaft in Gegenwart von 0,01 bis 0,2 Gew.-% eines Polymerisations-Inhibitors, bezogen auf die Gesamtmenge der Reaktanden, unter Normaldruck durchgeführt. Es ist aber auch möglich, das erfindungsgemässe Verfahren bei einem Unter- oder Überdruck durchzuführen. Ein geeigneter Inhibitor ist zum Beispiel 2,6-Di-tert. Butyl-4-methylphenol. Geeignet ist auch Luft, die in das Reaktionsgemisch eingeleitet wird. Das erfindungsgemässe Verfahren kann beispielsweise wie folgt durchgeführt werden:

Die Reaktanden werden in dem Lösungsmittel gelöst und unter Rühren mit dem Katalysator versetzt. Der zeitliche Verlauf der Umsetzung kann beispielsweise durch Messung der IR-Spektren verfolgt werden. Nach vollständiger Umsetzung der Isocyanatgruppen werden die Reaktionsprodukte durch Entfernen des Lösungsmittels isoliert. Eine vorherige Reinigung mit Hilfe von Adsorbentien, beispielsweise Aktivkohle, Bleicherde, Kieselgel oder Aluminiumoxid ist möglich.

Für die Anwendung als Monomere für polymere Zahnfüllmassen oder Beschichtungsmittel (Zahnlacke) im Dentalbereich können die erfindungsgemässen (Meth)-Acrylsäureester der Formel I mit an sich bekannten Monomeren gemischt werden, um beispielsweise die Viskosität dem Verwendungszweck anzupassen. Viskositäten im Bereich von 60 bis 10 000 mPas sind dabei bevorzugt. Dies ist dadurch erreicht, dass man den erfindungsgemässen Monomeren gegebenenfalls ein Comonomer niedrigerer Viskosität als Reaktivverdünner zumischt. Die erfindungsgemässen Verbindungen werden in der Mischung mit Comonomeren mit einem Anteil von ca. 30 bis ca. 90 Gew.-% eingesetzt, wobei ein Anteil von 50 bis 90 Gew.-% besonders bevorzugt ist.

Im Rahmen der vorliegenden Erfindung ist es ebenfalls bevorzugt, Mischungen verschiedener erfindungsgemässer (Meth)-Acrylsäureester einzusetzen.

Es ist auch möglich, Monomermischungen einzusetzen, die mehrere Comonomere als Reaktivverdünner enthalten.

Beispielsweise seien die folgenden Comonomere genannt:

Triethylenglykoldimethacrylat, Tetraethylenglykoldimethacrylat, 1,12-Dodecandioldimethacrylat, 1,6-Hexandioldimethacrylat, Diethylenglykoldimethacrylat, 2,2-Bis[p-(2'-hydroxy-3'-methacryloyloxypropoxy)phenyl]propan, 2,2-Bis[2'-methacryloyloxyethoxy)phenyl]propan, Trimethylolpropan-tri(meth)-acrylat, Bis-(Meth)acryloyloxyethoxymethyl)-tricyclo[5,2,1,$0^{2.6}$]decan (gemäss DE-OS-2 931 925 und 2 931 926) usw.

Insbesondere werden Comonomere bevorzugt, die bei 13 mbar einen Siedepunkt über 100 °C besitzen.

Die erfindungsgemässen (Meth)-Acrylsäureester lassen sich, gegebenenfalls in Mischung mit dem genannten Monomeren, mit an sich bekannten Methoden zu vernetzten Polymerisaten aushärten [G.M. Brauer, H. Argentar, Am. Chem. Soc., Symp. Ser. 212, S. 359–371 (1983)]. Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind:

Dibenzoylperoxid, Dilauroylperoxid und Di-4-Chlorbenzoylperoxid.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-PS-2 759 239 beschriebene N-Methyl-N-(2-methylcarbamoyloxypropyl)-3,5-dimethylanilin genannt.

Die Konzentrationen des Peroxids bzw. des Amins werden vorteilhaft so gewählt, dass sie 0,1 bis 5 Gew.-%, bevorzugt 0,5 bis 3 Gew.-%, bezogen auf die Monomermischung betragen. Die peroxid- bzw. aminhaltige Monomermischungen werden bis zur Anwendung getrennt gelagert.

Die erfindungsgemässen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die fotoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Fotoaktivatoren, wie N,N-Dimethylaminoethylmethacrylat, Triethanolamin, 4-N,N-Dimethylaminobenzolsulfonsäurebisallylamid. Die Durchführung des Fotopolymerisationsverfahrens ist beispielsweise in der DE-PS-3 135 115 beschrieben.

Neben den oben beschriebenen Initiatoren können den erfindungsgemässen (Meth)-Acrylsäureestern an sich für diesen Einsatzzweck bekannte Lichtschutzmittel und Polymerisations-Inhibitoren zugesetzt werden.

Die Lichtschutzmittel und der Polymerisations-Inhibitor werden jeweils im allgemeinen in einer Menge von 0,01 bis 0,50 Gew.-Teilen, bezogen auf 100 Gew.-Teile der Monomermischung eingesetzt. Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10 000 mPas besitzen, besonders vorteilhaft. Den die erfindungsgemässen Verbindungen der Formel I enthaltenden Monomermischungen können vorzugsweise anorganische Füllstoffe zugemischt werden. Beispielsweise seien Bergkristall, Quarzit, Kristobalit, Quarzglas, hochdisperse Kieselsäure, Aluminiumoxid und Glaskeramiken, beispielsweise Lanthan und Zirkon enthaltende Glaskeramiken (DE-OS-2 347 591) genannt.

Die anorganischen Füllstoffe werden zur Verbesserung des Verbundes zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden [E.P. Plueddemann, Progress in Organic coatings, 11, 297

bis 308 (1983)]. Bevorzugt wird 3-Methacryloyloxypropyl-trimethoxysilan eingesetzt.

Die Füllstoffe für die erfindungsgemässen Zahnfüllmassen weisen im allgemeinen einen mittleren Teilchendurchmesser von 0,01 bis 100 µm, vorzugsweise von 0,05 bis 50 µm auf, besonders bevorzugt 0,05 bis 5 µm. Es kann auch vorteilhaft sein, mehrere Füllstoffe nebeneinander einzusetzen, die einen voneinander verschiedenen Teilchendurchmesser besitzen.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verarbeitet.

Der Anteil der erfindungsgemässen (Meth)-Acrylsäureester in den Füllmassen beträgt im allgemeinen 5 bis 85 Gew.-%, bezogen auf die Füllmasse.

Beispiel 1

Herstellung von 1,2-Bis-(3-isocyanatophenyl)-1,1,2,2-tetrafluorethan

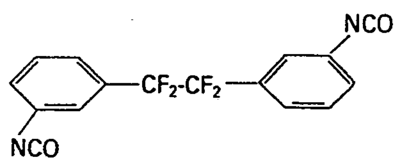

In 2,3 l Chlorbenzol werden bei ca. 0 °C 621 g Phosgen einkondensiert. Bei einer Temperatur von −10 °C bis +10 °C werden 353 g 1,2-Bis(3-aminophenyl)-1,1,2,2-tetrafluorethan, gelöst in 1,2 l Chlorbenzol, langsam zugegeben. Der Ansatz wird im Verlauf von vier Stunden bis 120 °C hochgeheizt. Ab etwa 80 °C wird weiter gasförmiges Phosgen eingeleitet. Bei 100 bis 110 °C wird die Lösung klar. Man leitet bei 120 °C noch 90 min lang Phosgen durch die Lösung. Anschliessend wird mit $CO_2$ das noch gelöste Phosgen ausgeblasen und dann der Ansatz durch Destillation aufgearbeitet. Man erhält 355 g 1,2-Bis-(3-Isocyanatophenyl)-1,1,2,2-tetrafluorethan, Kp: 145 bis 150 °C/0,1 mbar, Fp: 139 bis 140 °C (Reinheit lt. GC 97%).

Setzt man zur Phosgenierung ein ungereinigtes Diamin ein, wie man es durch Hydrierung der entsprechenden, nicht umkristallisierten Dinitroverbindung erhält, so bekommt man nach dem gleichen Verfahren der Basenphosgenierung wie oben das Diisocyanat mit einem Gehalt an von ca. 80%. Fp: 128 bis 135 °C.

Ein Diisocyanatgemisch des 1,2-Diphenyl-1,1,2,2-tetrafluorethans mit einem Gehalt von nur ca. 45% reinem 3,3′-Diisocyanat erhält man dann, wenn man aus dem Gemisch der entsprechenden Dinitroverbindungen die reine 3,4-Verbindung durch Umkristallisieren aus Toluol abtrennt und die so erhaltenen Mutterlaugen aufarbeitet, hydriert und wie oben beschrieben phosgiert.

Der Schmelzbereich des destillierten Diisocyanatgemisches ist bei 117 bis 127 °C.

Beispiel 2

(3,3′-Isomer der Verbindung 2 aus Tabelle 1)

Herstellung von

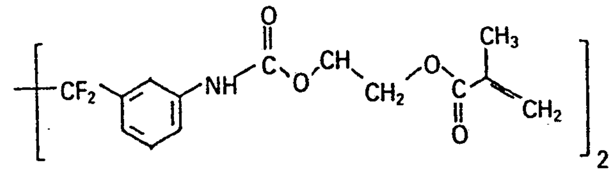

16,8 g (50 mMol) 1,2-Bis-(3-isocyanatophenyl)-1,1,2,2-tetrafluorethan aus Beispiel 1 werden in 100 ml Acetonitril suspendiert. 50 mg Dibutylzinndilaurat werden zugegeben. Nach Erwärmen auf 50 °C werden 13 g (100 mMol) 2-Hydroxyethylmethacrylat zugetropft. Zur Stabilisierung wird Luft durch das Reaktionsgemisch geleitet. Der Reaktionsverlauf wird IR-spektroskopisch bis zum Verschwinden der Absorptionsbande der Isocyanatgruppe verfolgt. Die Reaktionszeit beträgt etwa 36 Stunden, kann aber durch Erhöhung der Temperatur bzw. Verwendung von Zinn-(II)-octoat noch verkürzt werden.

Zur Aufarbeitung wird ein Teil des Lösungsmittels im Vakuum entfernt und die Suspension abfiltriert.

Der zurückbleibende Feststoff (17,5 g; 58,7%) besitzt aufgrund der $^1$H-NMR-Daten die erwartete Struktur. Schmelzpunkt: 160 °C.

Beispiel 3

3,3′-Isomer der Verbindung 3 aus Tabelle 1

Herstellung von

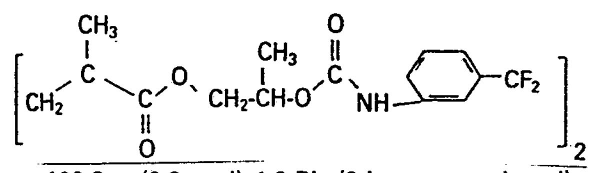

100,8 g (0,3 mol) 1,2-Bis-(3-Isocyanatophenyl)-1,1,2,2-tetrafluorethan werden in 500 ml Chloroform suspendiert. 1 g Dibutylzinndilaurat und 80 mg 2,5-Di-tert.-butyl-4-methylphenol werden zugesetzt. Der auf 50 °C bis 60 °C erwärmten Lösung werden 86,4 g (0,6 mol) 2-Hydroxypropyl-methacrylat tropfenweise hinzugefügt. Nach beendeter Reaktion liegt eine klare Lösung vor, die leicht gelb gefärbt ist. Man verrührt mit Aktivkohle und filtriert diese über Celite ab. Das Filtrat wird vom Lösungsmittel befreit. Die Ausbeute ist nahezu quantiativ. Das Produkt liegt als farblose viskose Flüssigkeit vor.

Die oben angegebene Struktur wird aufgrund der IR- und $^1$H-NMR-Spektren bestätigt.

Die Molmasse wurde osometrisch mit 625 ermittelt (ber. 624).

Beispiel 4

(Verbindung 3 aus Tabelle 1)

Geht man von einem Gemisch von 1,2-Bis-(3-isocyanatophenyl)-1,1,2,2-tetrafluorethan und 1,2-Bis-(4-isocyanatophenyl)-1,1,2,2-tetrafluorethan aus, entsteht in analoger Weise zu Beispiel 3 das meta/para-Isomerengemisch.

Beispiel 5
(Gemisch der isomeren Verbindungen 6 und 11 aus Tabelle 1)
Umsetzung von 1,2-Bis-(Isocyanatophenyl)-1,1,2,2-tetrafluorethan mit Bis-(methacryloxy)-hydroxypropan (Isomerengemisch).

67,2 g (200 mMol) eines Isomerengemisches von Bis-(3-isocyanatophenyl)-1,1,2,2-tetrafluorethan und Bis-(4-isocyanatophenyl)-1,1,2,2-tetrafluorethan werden in 200 ml Chloroform suspendiert und 0,4 g Dibutylzinndilaurat sowie 0,063 g 2,5-Di-tert.-butyl-4-methylphenol zugegeben. Die Mischung wird auf 50 °C erwärmt. Bei dieser Temperatur werden 91,2 g (400 mMol) Bis-(methacryloxy)-hydroxy-propan zugetropft.

Nach einer Reaktionszeit von 3 h ist die Umsetzung beendet. Die Reaktionsmischung wird wie in Beispiel 3 aufgearbeitet. Das Produkt ist eine farblose viskose Flüssigkeit.
Molmasse (Osmometr.) 787.

Anwendungsbeispiele:

Beispiel 6

Herstellung von Beschichtungslösungen

a) redoxhärtendes System
In einer Lösung aus des in Beispiel 3 genannten Monomers (70 T.) und Triethylenglykoldimethacrylat (30 T.) werden 1,94 Gew.-% Di-Benzoylperoxid gelöst (entsprechend 0,008 Mol Peroxid pro 100 g Monomermischung).

In einer zweiten, kein Peroxid enthaltenden, sonst aber gleich zusammengesetzten Mischung werden 2 Gew.-% N-Methyl-N-(2-Methylcarbamoyloxy-propyl)-3,5-dimethylanilin (DE-PS 2 759 239) gelöst.

Eine Mischung aus gleichen Teilen der beiden zuvor beschriebenen Lösungen härtet in 2 bis 3 min aus.

Die mechanischen Eigenschaften verschiedener Monomere sind in der Tabelle 2 aufgeführt.

b) lichthärtendes System
In einer Monomermischung der unter a) beschriebenen Zusammensetzung (ohne Peroxid) werden 0,5 Gew.-% 4-N,N-Di-methylaminobenzolsulfonsäure-bis-allylamid, 0,125 Gew.-% Benzildimethylketal, 0,2 Gew.-% Bicyclo[2,2,1]-1,7,7-trimethyl-heptan-2,3-dion-(2,3-Bornandion) gelöst.

Beim Belichten mit einer Dentallampe härtet die Flüssigkeit aus (Belichtungszeit 40 sek).

Beispiel 7
Beispiel 6 wird unter Verwendung des Monomers aus Beispiel 5 wiederholt, wobei jedoch 32,8 Gewichtsteile Triethylenglykoldimethacrylat verwendet werden.

Beispiel 8
Prüfung der Beschichtungslösungen aus den Beispielen 6 und 7

a) Wallace-Härteprüfung
Probenherstellung für redoxhärtende Beschichtungslösung:
Die beiden in Beispiel 6a) beschriebenen, jeweils Amin oder Peroxid enthaltenden Lösungen werden im Gewichtsverhältnis 1:1 30 sek lang gemischt und in eine Ringform gegossen, die mit einer Metallplatte (mit Polyamidfolie überzogen) abgedeckt wird. Nach der Aushärtung der Probe wird eine zweite, mit Polyamidfolie bedeckte Metallplatte aufgelegt. Die Metallplatten werden mit Hilfe einer Zwinge zusammengepresst. Die Form wird 135 min in ein Wasserbad von 37 °C gegeben. Danach werden die Probekörper entnommen und beidseitig mit Siliciumcarbidpapier (1000) beschliffen.
Probenherstellung für lichthärtende Beschichtungslösung:
Die in Beispiel 6b) beschriebene Lösung wird in eine mit einer Polyamidfolie abgedeckte Ringform gegossen und nach Auflegen einer zweiten Folie durch Bestrahlen (beidseitig 60 sek) mit einer Dentallampe ausgehärtet. Danach wird wie beim redoxgehärteten Probekörper verfahren.
Die Messung der Wallace-Eindringtiefe erfolgt im Zeitraum von 15 bis 45 min nach Aushärtung der Proben. Eine Vickerspyramide wird dabei 15 sek lang unter einer Vorlast von 1 g und dann 60 sek lang unter einer Hauptlast von 100 g aufgedrückt.
Eine Eindringtiefe des Diamanten unter Einwirkung der Hauptlast wird als Wallace-Eindringtiefe ($H_w$ 100/60) in µm registriert. Es werden für die Beurteilung einer Probe jeweils 5 Messungen herangezogen.

b) Prüfung der mechanischen Eigenschaften
Das Beschichtungsmittel aus Beispiel 6 wurde gemäss der DIN 13 922 der Prüfung von Biegefestigkeit und Biegemodul unterzogen.

Beispiel 9
Beispiel 8 wurde unter Verwendung des Beschichtungmittels aus Beispiel 7 wiederholt.
Die Messergebnisse aus Beispiel 8 und Beispiel 9 sind in Tabelle 2 zusammengefasst.

Tabelle 2
Prüfung der Beschichtungslösungen aus den Beispielen 6 und 7
(wenn nicht anders vermerkt, entspricht die Polymerisations-Aktivierung der von Beispiel 6 und 7)

| Zusammensetzung des Beschichtungsmittels (Gewichtsteile) | | | Biegefestigkeit [N/mm²] | Biegemodul [N/mm²] | Wallace-Eindringtiefe [mm] |
|---|---|---|---|---|---|
| A) | 70 T. | Monomer aus Beispiel 3 | | | |
| | 30 T. | TEGDMA* | | | |
| | | redoxhärtend 1,6% Peroxid: 1,7% Amin | 95,3 | 5982 | – |
| | | redoxhärtend | 100,0 | | 0,0161+0,0003 |
| | | lichthärtend | | | 0,0140+0,0007 |
| B) | 67,2 T. | Monomer aus Beispiel 5 | | | |
| | 32,8 T. | TEGDMA* | | | |
| | | redoxhärtend | | | 0,0156+0,0017 |
| | | lichthärtend | 90,4 | 2115 | 0,0149+0,0011 |
| C) | 56,2 T. | Bis-GMA (Vergleich) | | | |
| | 32,8 T. | TEGHDMA* | | | |
| | | redoxhärtend | | | 0,0169+0,0011 |
| | | lichthärtend | 71,2 | 1995 | 0,0152+0,0018 |

* TEGDMA = Triethylenglykoldimethacrylat
Bis-GMA = Bisphenol A-diglycidyldimethacrylat
(=2,2-Bis[p-(2′-hydroxy-3′-methacryloyloxy)-propoxyphenyl]propan

Beispiel 10

Herstellung einer redoxhärtenden Zahnfüllmasse

Aminpaste: In einer Mischung von 70 Gewichtsteilen der erfindungsgemässen Verbindung aus Beispiel 3 und 30 Gewichtsteilen Triethylenglykoldimethacrylat werden 2,0 Gew.-% N-Methyl-N-(2-Methylcarbamoyloxypropyl-3,5-dimethylanilin gelöst. 5 g dieser Lösung werden mit 15 g einer handelsüblichen Glaskeramik mit einem mittleren Teilchendurchmesser von 4 µm, die mit 3-Methacryloyloxypropyl-trimethoxysilan silanisiert wurden, zu einer Paste verarbeitet.

Peroxidpaste: In einer Mischung von 70 Gewichtsteilen der erfindungsgemässen Verbindung aus Beispiel 3 und 30 Gewichtsteilen Triethylenglykoldimethacrylat werden 1,94 Gew.-% Dibenzoylperoxid gelöst. 5 g dieser Lösung werden mit 15 g einer handelsüblichen Glaskeramik mit einem mittleren Teilchendurchmesser von 4 µm, die mit 3-Methacryloyloxypropyl-trimethoxysilan silanisiert wurden, zu einer Paste verarbeitet.

Eine Mischung von gleichen Teilen Aminpaste und Peroxidpaste härtet innerhalb von 2 bis 3 min aus.

Beispiel 11

Herstellung eines lichthärtenden Zahnfüllmaterials

In einer Mischung aus 70 Gewichtsteilen Monomer aus Beispiel 3 und 30 Gewichtsteilen Triethylenglykoldimethacrylat werden 0,2 Gew.-% 2,3-Bornandion, 0,125 Gew.-% Benzildimethylketal und 0,5 Gew.-% 4-N,N-Dimethylaminobenzolsulfonsäure-bis-allylamid gelöst.

5 g dieser Lösung werden mit 15 g des in Beispiel 9 beschriebenen Füllstoffs zu einer Paste verarbeitet (74% Füllstoffgehalt).

Die Aushärtung erfolgt durch Belichtung mit einer Dentallampe der Fa. Kulzer. Bei einer Belichtungszeit von 40 sek beträgt die Aushärtungstiefe 6 mm.

Beispiel 12

Beispiel 10 wird unter Verwendung des Monomers aus Beispiel 5 wiederholt, wobei jedoch 32,8 Gewichtsteile Triethylenglykoldimethacrylat verwendet werden.

Beispiel 13

Prüfung der Dentalmassen aus den Beispielen 10 und 11

Die Prüfung von Biegefestigkeit und Biegemodul wird gemäss der DIN 13922 durchgeführt. Die Ergebnisse sind in Tabelle 3 zusammengefasst.

Tabelle 3
Mechanische Eigenschaften von Dentalmassen
(Lichtaktivierung gemäss Beispielen 10 und 11)

| Monomermischung | Füllstoffgehalt | Biegefestigkeit [N/mm²] | Biegemodul [N/mm²] |
|---|---|---|---|
| 70 T. Monomer aus Beispiel 3<br>30 T. TEGDMA* | 75% Glaskeramik | 143,5 | 12890 |
| 67,2 T. Monomer aus Beispiel 5<br>32,8 T. TEGDMA* | 75% Glaskeramik | 128,7 | 13250 |

* TEGDMA = Triethylenglykoldimethacrylat

Beispiel 14
Messung von Festkörperoberflächenspannungen

An durch lichtinduzierte Polymerisation ausgehärteten Beschichtungsmitteln der Beispiele 6 und 7 wurden Messungen der Oberflächenspannung durchgteführt. Mittels eines Video-Systems wurde das dynamische Benetzungsvehalten von Flüssigkeiten auf den Festkörperoberflächen bestimmt. Die Oberflächenspannungen wurden aus den Anfangsrandwinkeln von 5 Prüfflüssigkeiten berechnet. Die Ergebnisse sind in der Tabelle 4 zusammengestellt.

Tabelle 4
Messung der Festkörperoberflächenspannungen

| Monomer aus Beispiel | Verhältnis Monomer zu TEGDMA* | Gesamt [mN/m] | unpolarer Anteil [mN/m] | polarer Anteil [mN/m] | polarer Anteil [%] |
|---|---|---|---|---|---|
| 3 | 60/40 | 39,7 | 30,2 | 9,5 | 23,9 |
| 3 | 77/23 | 39,6 | 33,8 | 5,8 | 14,6 |
| 5 | 67,2/32,8 | 40,7 | 35,2 | 5,5 | 13,5 |
| Vergleich (Bis-GMA) | 67,2/32,8 | 42,2 | 28,7 | 13,5 | 32,0 |

* TEGDMA = Triethylenglykoldimethacrylat

**Patentansprüche**

1. (Meth)-Acrylsäureester der Formel

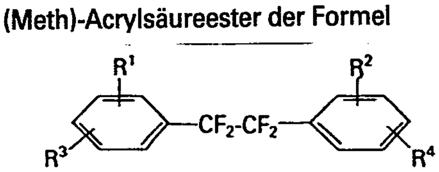

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

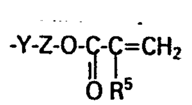

stehen, wobei

$R^5$ für Wasserstoff oder Methyl steht,

Y ein zweibindiges Brückenglied aus der Gruppe

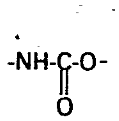 oder 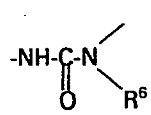 und

wobei

$R^6$ für Wasserstoff, Niederalkyl ($C_1$ bis $C_6$) oder Phenyl steht, bedeutet, und

Z eine geradkettige oder verzweigte Kohlenwasserstoffkette mit 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist.

2. (Meth)-Acrylsäureester nach Anspruch 1 der Formel

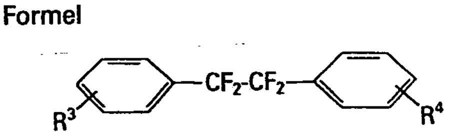 (II)

in der

$R^3$ und $R^4$ gleich oder verschieden sind und für einen der Reste

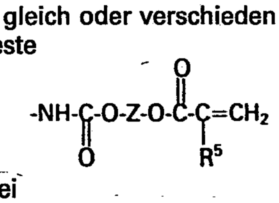

stehen, wobei

$R^5$ Wasserstoff oder Methyl bedeutet, und

Z eine geradkettige oder verzweigte Kohlenstoffkette von 2 bis 10 Kolenstoffatomen bedeutet, die gegebenenfalls Sauerstoffbrücken enthalten

kann und gegebenenfalls durch 1 bis 4 Acrylat- oder Methacrylatreste substituiert ist.

3. Verfahren zur Herstellung von (Meth)-Acrylsäureestern der Formel

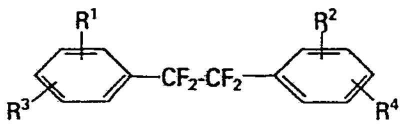

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

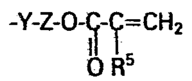

stehen, wobei

$R^5$ für Wasserstoff oder Methyl steht,

Y ein zweibindiges Brückenglied aus der Gruppe

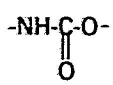 oder 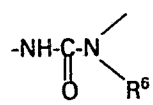

wobei

$R^6$ für Wasserstoff, Niederalkyl ($C_1$ bis $C_6$) oder Phenyl steht, bedeutet, und

Z eine geradkettige oder verzweigte Kohlenwasserstoffkette mit 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist, dadurch gekennzeichnet, dass man einen 1,2-Bis-(isocyanatophenyl)-tetrafluorethan der Formel

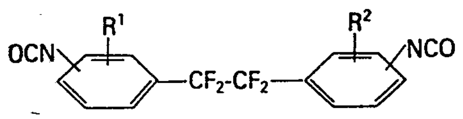

in der

$R^1$ und $R^2$ gleich oder verschieden sind und Wasserstoff, Chlor, Fluor oder einen $C_1$- bis $C_4$-Alkylrest bedeuten,

mit (Meth)-Acrylsäure-Derivaten der Formel

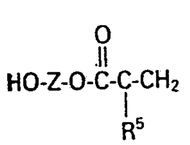 oder 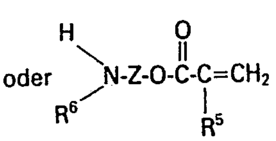

in denen

Z eine geradkettige oder verzweigte Kohlenstoffkette von 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist,

$R^5$ Wasserstoff oder Methyl und

$R^6$ Wasserstoff, Niederalkyl ($C_1$ bis $C_6$) oder Phenyl bedeuten,

in einem inerten Lösungsmittel in Gegenwart eines Katalysators im Temperaturbereich von 20 bis 100 °C umsetzt.

4. Polymerisat aus (Meth)-Acrylsäureester der Formel

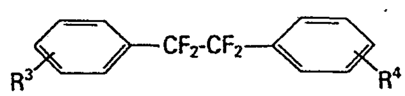

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

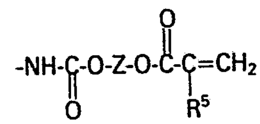

stehen, wobei

$R^5$ für Wasserstoff oder Methyl steht,

Y ein zweibindiges Brückenglied aus der Gruppe

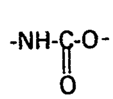 oder 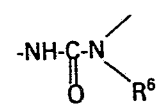 und

wobei

$R^6$ für Wasserstoff, Niederalkyl ($C_1$ bis $C_6$) oder Phenyl steht, bedeutet, und

Z eine geradkettige oder verzweigte Kohlenstoffkette von 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist.

5. Verwendung von (Meth)-Acrylsäureestern der Formel

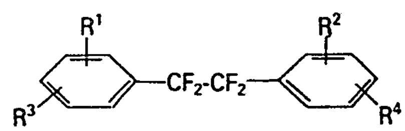

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

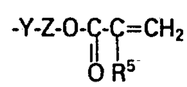

stehen, wobei

$R^5$ für Wasserstoff oder Methyl steht,

Y ein zweibindiges Brückenglied aus der Gruppe

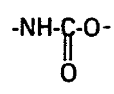 oder 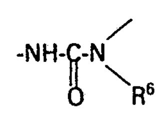 und

wobei

$R^6$ für Wasserstoff, Niederalkyl ($C_1$ bis $C_6$) oder Phenyl steht, bedeutet, und

Z eine geradkettige oder verzweigte Kohlenwasserstoffkette mit 2 bis 10 Kohlenstoffatomen bedeutet, die gegebenenfalls Sauerstoffbrücken enthalten kann und gegebenenfalls durch 1 bis 4

Acrylat- oder Methacrylatreste substituiert ist, im Dentalbereich.

6. Verwendung von (Meth)-Acrylsäureestern der Formel

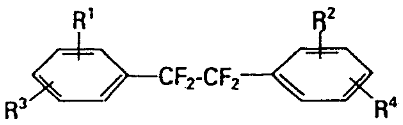

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

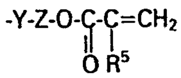

stehen, wobei

$R^5$ für Wasserstoff oder Methyl steht,

Y ein zweibindiges Brückenglied aus der Gruppe

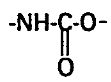 oder 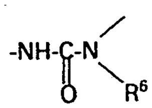

wobei

$R^6$ für Wasserstoff, Niederalkyl ($C_1$ bis $C_6$) oder Phenyl steht, bedeutet, und

Z eine geradkettige oder verzweigte Kohlenwasserstoffkette mit 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist, bei der Herstellung von Zahnfüllmassen und Zahnbeschichtungsmitteln.

7. Verwendung nach Anspruch 5, dadurch gekennzeichnet, dass die (Meth)-Acrylsäureester in Zahnfüllmassen eingesetzt werden.

8. Verwendung nach Anspruch 5, dadurch gekennzeichnet, dass die (Meth)-Acrylsäureester in Beschichtungsmitteln für Zähne eingesetzt werden.

9. Zahnfüllmassen, dadurch gekennzeichnet, dass sie (Meth)-Acrylsäureester der Formel

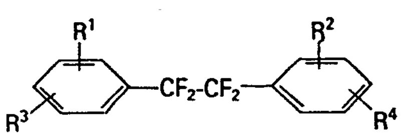

enthalten, in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für einen der Reste

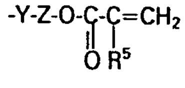

stehen, wobei

$R^5$ für Wasserstoff oder Methyl steht,

Y ein zweibindiges Brückenglied aus der Gruppe

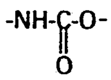 oder 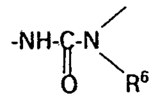

wobei

$R^6$ für Wasserstoff, Niederalkyl ($C_1$ bis $C_6$) oder Phenyl steht, bedeutet, und

Z eine geradkettige oder verzweigte Kohlenstoffkette von 2 bis 10 Kohlenstoffatomen bedeutet, die gegebenenfalls Sauerstoffbrücken enthalten kann und gegebenenfalls durch 1 bis 4 Acrylat- oder Methacrylatreste substituiert ist.

**Revendications**

1. Esters d'acide (méth)acrylique de formule

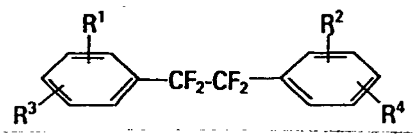

dans laquelle

$R^1$ et $R^2$ sont identiques ou différents et représentent l'hydrogène, le chlore, le fluor ou un radical alkyle $C_1$–$C_4$ et

$R^3$ et $R^4$ sont identiques ou différents et représentent un des groupements

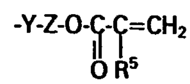

où

$R^5$ représente l'hydrogène ou un groupe méthyle,

Y représente un chaînon bivalent choisi parmi les groupes

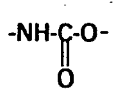 où 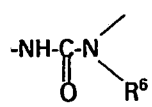

$R^6$ représente l'hydrogène, un groupe alkyle inférieur ($C_1$–$C_6$) ou phényle, et représente und chaîne hydrocarbonée droite ou ramifiée comportant 2 à 10 atomes de carbone, qui peut éventuellement contenir des ponts d'oxygène et éventuellement être substituée par 1 à 4 groupements acrylate ou méthacrylate.

2. Esters d'acide (méth)acrylique selon la revendication 1, de formule

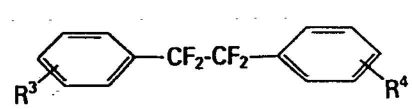

dans laquelle

$R^3$ et $R^4$ sont identiques ou différents et représentent un des groupements

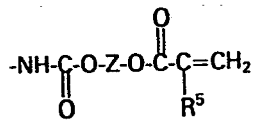

où

$R^5$ représente l'hydrogène ou un groupe méthyle, et

Z représente une chaîne hydrocarbonée droite ou ramifiée comportant 2 à 10 atomes de carbone, pouvant éventuellement contenir des ponts oxygène et étant éventuellement substituée par 1 à 4 groupements acrylate ou méthacrylate.

3. Procédé pour la fabrication d'esters d'acide (méth)acrylique de formule

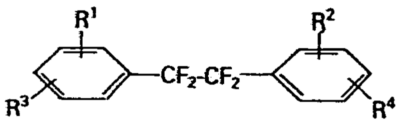

dans laquelle

$R^1$ et $R^2$ sont identiques ou différents et représentent l'hydrogène, le chlore, le fluor ou un radical alkyle $C_1$–$C_4$ et

$R^3$ et $R^4$ sont identiques ou différents et représentent un des groupements

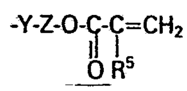

où

$R^5$ représente l'hydrogène ou un groupe méthyle,

Y représente un chaînon bivalent choisi parmi les groupes

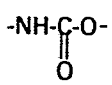 où 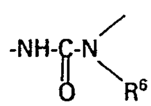

$R^6$ représente l'hydrogène, un groupe alkyle inférieur ($C_1$–$C_6$) ou phényle, et

Z représente une chaîne hydrocarbonée droite ou ramifiée comportant 2 à 10 atomes de carbone, qui peut éventuellement contenir des ponts d'oxygène et éventuellement être substituée par 1 à 4 groupements acrylate ou méthacrylate,

caractérisé en ce que l'on fait réagir un 1,2-bis-(isocyanatophényl)-tétrafluoréthane de formule

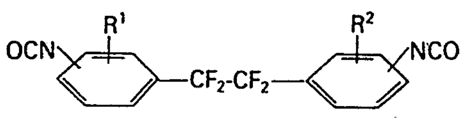

dans laquelle

$R^1$ et $R^2$ sont identiques ou différents et représentent l'hydrogène, le chlore, le fluor ou un groupement alkyle $C_1$–$C_4$,

avec des dérivés d'acide (méth)acrylique de formule

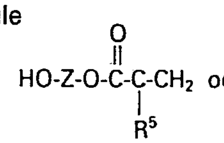 oder 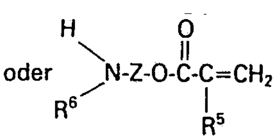

dans laquelle

Z représente une chaîne hydrocarbonée droite ou remifiée comportant 2 à 10 atomes de carbone, qui peut éventuellement contenir des ponts oxygène et éventuellement être substituée par 1 à 4 groupements acrylate ou méthacrylate,

$R^5$ représente l'hydrogène ou un groupe méthyle, et

$R^6$ représente l'hydrogène, un groupe alkyle inférieur ($C_1$–$C_6$) ou phényle,

dans un solvant inerte et en présence d'un catalyseur, dans l'intervalle de températures de 20 à 100°C.

4. Polymère d'ester d'acide méthacrylique de formule

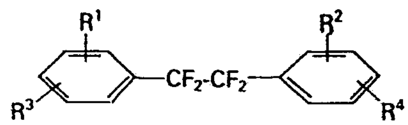

dans laquelle

$R^1$ et $R^2$ sont identiques ou différents et représentent l'hydrogène, le chlore, le fluor ou un radical alkyle $C_1$–$C_4$ et

$R^3$ et $R^4$ sont identiques ou différents et représentent un des groupements

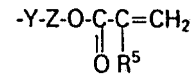

où

$R^5$ représente l'hydrogène ou un groupe méthyle,

Y représente un chaînon bivalent choisi parmi les groupes

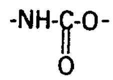 où 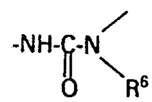

$R^6$ représente l'hydrogène, un groupe alkyle inférieur ($C_1$–$C_6$) ou phényle, et

Z représente une chaîne hydrocarbonée droite ou ramifiée comportant 2 à 10 atomes de carbone, qui peut éventuellement contenir des ponts d'oxygène et éventuellement être substituée par 1 à 4 groupements acrylate ou méthacrylate.

5. Utilisation des esters d'acide (méth)acrylique de formule

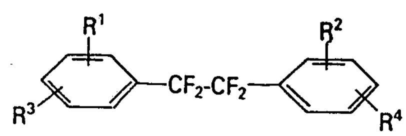

dans laquelle

$R^1$ et $R^2$ sont identiques ou différents et représentent l'hydrogène, le chlore, le fluor ou un radical alkyle $C_1$–$C_4$ et

$R^3$ et $R^4$ sont identiques ou différents et représentent un des groupements

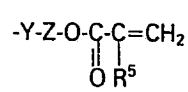

où

$R^5$ représente l'hydrogène ou un groupe méthyle,

Y représente un chaînon bivalent choisi parmi les groupes

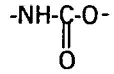 où 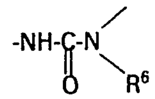

$R^6$ représente l'hydrogène, un groupe alkyle inférieur ($C_1$–$C_6$) ou phényle, et

Z représente une chaîne hydrocarbonée droite ou ramifiée comportant 2 à 10 atomes de carbone, qui peut éventuellement contenir des ponts d'oxygène et éventuellement être substituée par 1 à 4 groupements acrylate ou méthacrylate,

en technique dentaire.

6. Utilisation d'esters d'acide (méth)acrylique de formule

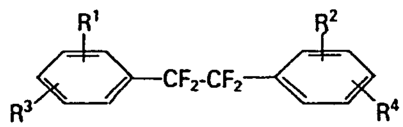

dans laquelle

$R^1$ et $R^2$ sont identiques ou différents et représentent l'hydrogène, le chlore, le fluor ou un radical alkyle $C_1$–$C_4$ et

$R^3$ et $R^4$ sont identiques ou différents et représentent un des groupements

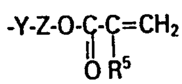

où

$R^5$ représente l'hydrogène ou un groupe méthyle,

Y représente un chaînon bivalent choisi parmi les groupes

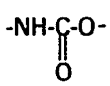 où 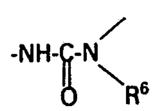

$R^6$ représente l'hydrogène, un groupe alkyle inférieur ($C_1$–$C_6$) ou phényle, et

Z représente une chaîne hydrocarbonée droite ou ramifiée comportant 2 à 10 atomes de carbone, qui peut éventuellement contenir des ponts d'oxygène et éventuellement être substituée par 1 à 4 groupements acrylate ou méthacrylate,

dans la fabrication de masses d'oburation dentaire et de produits pour revêtements dentaires.

7. Utilisation selon la revendication 5, caractérisée en ce que les esters d'acide (méth)acrylique sont utilisés dans des masses d'obturation dentaire.

8. Utilisation selon la revendication 5, caractérisée en ce que les esters d'acide (méth)acrylique sont utilisés dans des produits de revêtements pour dents.

9. Masses d'obturation dentaire, caractérisée en ce qu'elles contiennent des esters d'acide (méth)-acrylique de formule

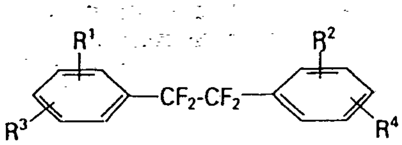

dans laquelle

$R^1$ et $R^2$ sont identiques ou différents et représent l'hydrogène, le chlore, le fluor ou un radical alkyle $C_1$–$C_4$ et

$R^3$ et $R^4$ sont identiques ou différents et représentent un des groupements

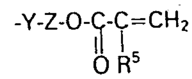

où

$R^5$ représente l'hydrogène ou un groupe méthyle,

Y représente un chaînon à bivalent choisi parmi les groupes

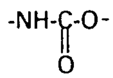 où 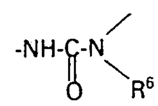

$R^6$ représente l'hydrogène, un groupe alkyle inférieur ($C_1$–$C_6$) ou phényle, et représente une chaîne hydrocarbonée droite ou ramifiée comportant 2 à 10 atomes de carbone, qui peut éventuellement contenir des ponts d'oxygène et éventuellement être substituée par 1 à 4 groupements acrylate ou méthacrylate.

## Claims

1. (Meth)-acrylic acid esters of the formula

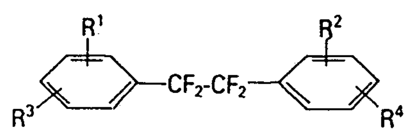

in which

$R^1$ and $R^2$ are identical or different and denote hydrogen, chlorine, fluorine or a $C_1$- to $C_4$-alkyl radical and

$R^3$ and $R^4$ are identical or different and represent the radical

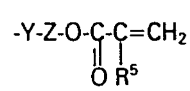

wherein

$R^5$ represents hydrogen or methyl,

Y denotes a divalent bridge member from the group

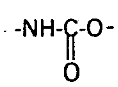 or 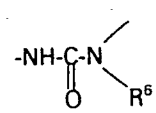

wherein

$R^6$ represents hydrogen, lower alkyl ($C_1$ to $C_6$) or phenyl, and

Z denotes a straight-chain or branched hydrocarbon chain which has 2 to 10 carbon atoms, can optionally contain oxygen bridges and is optionally substituted by 1 to 4 acrylate or methacrylate radicals.

2. (Meth)-acrylic acid esters according to claim 1, of the formula

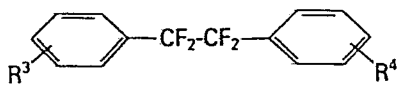

in which

$R^3$ and $R^4$ are identical or different and represent the radical

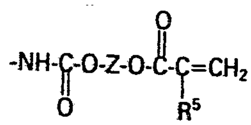

wherein

$R^5$ denotes hydrogen or methyl and

Z denotes a straight-chain or branched hydrocarbon chain which has 2 to 10 carbon atoms, can optionally contain oxygen bridges and is optionally substituted by 1 to 4 acrylate or methacrylate radicals.

3. Process for the preparation of (meth)-acrylic acid esters of the formula

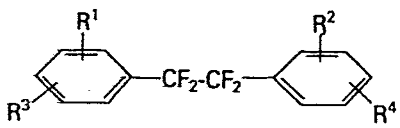

in which

$R^1$ and $R^2$ are identical or different and denote hydrogen, chlorine, fluorine or a $C_1$- to $C_4$-alkyl radical and

$R^3$ and $R^4$ are identical or different and represent the radical

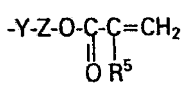

wherein

$R^5$ represents hydrogen or methyl,

Y denotes a divalent bridge member from the group

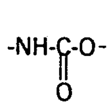 or 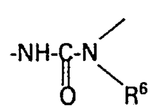

wherein

$R^6$ represents hydrogen, lower alkyl ($C_1$ to $C_6$) or phenyl, and

Z denotes a straight-chain or branched hydrocarbon chain which has 2 to 10 carbon atoms, can optionally contain oxygen bridges and is optionally substituted by 1 to 4 acrylate or methacrylate radicals,

characterized in that a 1,2-bis-(isocyanatophenyl)-tetrafluoroethane of the formula

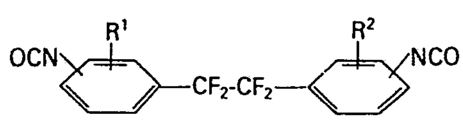

in which

$R^1$ and $R^2$ are identical or different and denote hydrogen, chlorine, fluorine or a $C_1$- to $C_4$-alkyl radical,

is reacted with (meth)-acrylic acid derivatives of the formula

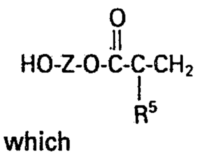 or 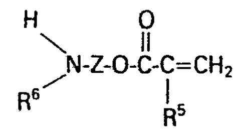

in which

Z denotes a straight-chain or branched hydrocarbon chain which has 2 to 10 carbon atoms, can optionally contain oxygen bridged and is optionally substituted by 1 to 4 acrylate or methacrylate radicals,

$R^5$ denotes hydrogen or methyl and

$R^6$ denotes hydrogen, lower alkyl ($C_1$ to $C_6$) or phenyl, in an inert solvent in the presence of a catalyst in the temperature range from 20 to 100 °C.

4. Polymer of (meth)-acrylic acid ester of the formula

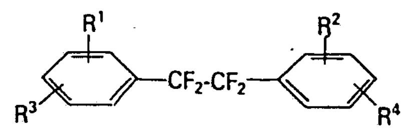

in which

$R^1$ and $R^2$ are identical or different and denote hydrogen, chlorine, fluorine or a $C_1$- to $C_4$-alkyl radical and

$R^3$ and $R^4$ are identical or different and represent the radical

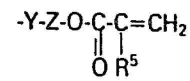

wherein

$R^5$ represents hydrogen or methyl,

Y denotes a divalent bridge member from the group

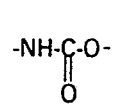 or 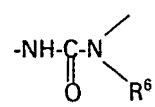

wherein

$R^6$ represents hydrogen, lower alkyl ($C_1$–$C_6$) or phenyl, and

Z denotes a straight-chain or branched hydrocarbon chain which has 2 to 10 carbon atoms, can optionally contain oxygen bridges and is optionally substituted by 1 to 4 acrylate or methacrylate radicals.

5. Use of (meth)-acrylic acid esters of the formula

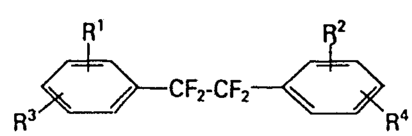

in which

$R^1$ and $R^2$ are identical or different and denote hydrogen, chlorine, fluorine or a $C_1$- to $C_4$-alkyl radical and

$R^3$ and $R^4$ are identical or different and represent the radical

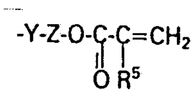

wherein

$R^5$ represents hydrogen or methyl,

Y denotes a divalent bridge member from the group

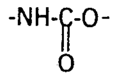 or 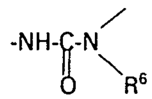

wherein

$R_6$ represents hydrogen, lower alkyl ($C_1$ to $C_6$) or phenyl, and

Z denotes a straight-chain or branched hydrocarbon chain which has 2 to 10 carbon atoms, can optionally contain oxygen bridges and is optionally substituted by 1 to 4 acrylate or methacrylate radicals, in the dental field.

6. Use of (meth)-acrylic acid esters of the formula

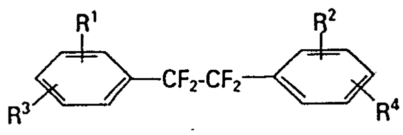

in which

$R^1$ and $R^2$ are identical or different and denote hydrogen, chlorine, fluorine or a $C_1$- to $C_4$-alkyl radical and

$R^3$ and $R^4$ are identical or different and represent the radical

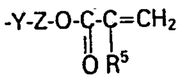

wherein

$R^5$ represents hydrogen or methyl,

Y denotes a divalent bridge member from the group

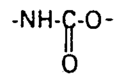 or 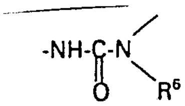

wherein

$R^6$ represents hydrogen, lower alkyl ($C_1$ to $C_6$) or phenyl, and

Z denotes a straight-chain or branched hydrocarbon chain which has 2 to 10 carbon atoms, can optionally contain oxygen bridges and is optionally substituted by 1 to 4 acrylate or methacrylate radicals,

in the preparation of dental filling compositions and coating agents for teeth.

7. Use according to claim 5, characterized in that the (meth)-acrylic acid esters are employed in dental filling compositions.

8. Use according to claim 5, characterized in that the (meth)-acrylic acid esters are employed in coating agents for teeth.

9. Dental filling compositions, characterized in that they contain (meth)-acrylic acid esters of the formula

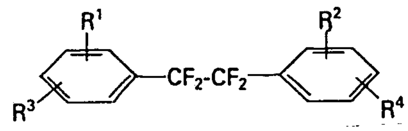

in which

$R^1$ and $R^2$ are identical or different and denote hydrogen, chlorine, fluorine or a $C_1$- to $C_4$-alkyl radical and

$R^3$ and $R^4$ are identical or different and represent the radical

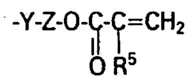

wherein

$R^5$ represents hydrogen or methyl,

Y denotes a divalent bridge member from the group

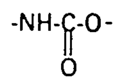 or 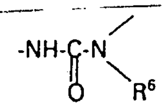

wherein

$R^6$ represents hydrogen, lower alkyl ($C_1$ to $C_6$) or phenyl, and

Z denotes a straight-chain or branched hydrocarbon chain which has 2 to 10 carbon atoms, can optionally contain oxygen bridges and is optionally substituted by 1 to 4 acrylate or methacrylate radicals.